# EUROPEAN PATENT SPECIFICATION

(11) **EP 2 217 703 B1**
(45) Date of publication and mention of the grant of the patent: **01.03.2017**
(21) Application number: 08853358.3
(22) Date of filing: 25.11.2008
(51) Int. Cl.: C12N 15/10, C07K 1/14

(54) **METHOD FOR ISOLATION OF GENOMIC DNA, RNA AND PROTEINS FROM A SINGLE SAMPLE**
VERFAHREN ZUR ISOLIERUNG VON GENOMISCHER DNA, RNA UND PROTEINEN AUS EINER EINZELPROBE
PROCÉDÉ D'ISOLATION D'ADN GÉNOMIQUE, D'ARN ET DE PROTÉINES, À PARTIR D'UN ÉCHANTILLON UNIQUE

(30) Priority: 30.11.2007 US 991337 P; 17.09.2008 US 97604 P
(43) Date of publication of application: 18.08.2010
(73) Proprietor: GE Healthcare Bio-Sciences Corp., Piscataway, NJ 08855 (US)
(72) Inventor: JIANG, Miao, Cedar Knolls New Jersey 07927 (US); BRIGGS, Mark S., Cardiff, South Glamorgan CF3 5UX (GB); DHULIPALA, Rohini, Kendall Park New Jersey 08824 (US); CAI, Yuyang Christine, Cranbury New Jersey 08512 (US); BRUNO, Renee E., Union New Jersey 07083 (US)
(74) Representative: Aldenbäck, Ulla Christina
(86) International application number: PCT/US2008/084616
(87) International publication number: WO 2009/070558

(56) References cited:
- EP-A- 1 529 841
- US-A1- 2002 192 667
- QIAGEN: "AllProtect and AllPrep" SCIENCE, vol. 317, 17 August 2007 (2007-08-17), page 965, XP002516513
- QIAGEN: "AllPrep DNA/RNA/Protein Mini Handbook" QIAGEN HANDBOOKS, [Online] December 2007 (2007-12), pages 1-60, XP002516514 Retrieved from the Internet: URL:www.qiagen.com> [retrieved on 2009-02-23]
- QIAGEN: "AllPrep RNA/Protein Hanbook" QIAGEN HANDBOOKS, [Online] January 2006 (2006-01), page 1-32, XP002516515 Retrieved from the Internet: URL:www.qiagen.com> [retrieved on 2009-02-23]
- QIAGEN: "AllPrep DNA/RNA Mini Handbook" QIAGEN HANDBOOKS, [Online] November 2005 (2005-11), page 1-56, XP002516516 Retrieved from the Internet: URL:www.qiagen.com> [retrieved on 2009-02-23]
- P. Muller: "Glossary of terms used in physical organic chemistry (IUPAC Recommendations 1994)", Pure and Applied Chemistry, vol. 66, no. 5, 1 January 1994 (1994-01-01), pages 1077-1184, XP055049175, ISSN: 0033-4545, DOI: 10.1351/pac199466051077

## Description

### Field of the Invention

This invention relates to methods for the isolation of genomic DNA, total RNA and protein. More specifically, it relates to a simple and rapid system and method for the extraction and purification of genomic DNA, total RNA and protein from a single sample.

### Background of the Invention

The last three decades has seen considerable effort in the development of improved methods for the isolation and purification of nucleic acids and proteins from biological sources. This has been due mainly to the increasing applications of nucleic acids and proteins in the medical and biological sciences. Genomic DNA isolated from blood, tissue or cultured cells has several applications, which include PCR, sequencing, genotyping, hybridization and Southern Blotting. Plasmid DNA has been utilized in sequencing, PCR, in the development of vaccines and in gene therapy. Isolated RNA has a variety of downstream applications, including *in vitro* translation, cDNA synthesis, RT-PCR and for microarray gene expression analysis. In the protein field, identification of proteins by Western Blotting has become an important tool in studying gene expression in basic research and identification of specific proteins for diagnostic purposes, as exemplified by viral protein detection.

The analysis and *in vitro* manipulation of nucleic acids and proteins is typically preceded by an isolation step in order to free the samples from unwanted contaminants which may interfere with subsequent processing procedures. For the vast majority of procedures in both research and diagnostic molecular biology, extracted nucleic acids and proteins are required as the first step.

The increased use of RNA, DNA and proteins has created a need for fast, simple and reliable methods and reagents for isolating DNA, RNA and proteins. In many applications, collecting the biological material sample and subsequent analysis thereof would be substantially simplified if the three cellular components (RNA, DNA and proteins) could be simultaneously isolated from a single sample. The simultaneous isolation is especially important when the sample size is so small, such as in biopsy, that it precludes its separation into smaller samples to perform separate isolation protocols for DNA, RNA and proteins.

Additionally, all three levels, DNA, RNA and protein, provide information that is valuable for different reasons. The DNA or genotype gives important information about genetic pre-dispositions and acquired mutations/local rearrangements. Both mRNA and protein profiles generate "molecular portraits" of a biological state/stage or disease, and may also be used for staging and monitoring of the disease development and treatment. As opposed to the DNA, both mRNA and protein profiles represent "snap shots" of the cell's biology, since they are continuously changing in response to the surrounding environment. Due to regulatory mechanisms acting both at the transcriptional, translational and post-translational levels, mRNA and protein levels do not always correlate. It is therefore crucial to study both mRNA and protein from the same sample.

Thus, as mentioned above, there is however not necessarily a 1:1 correlation between mRNA and protein levels. If protein levels and corresponding mRNA levels are compared, then for every protein for which the ratio of mRNA and protein is not 1:1 then this protein is subject to some form of interesting post transcriptional and/or post translational regulation. mRNA/protein ratios for specific genes are often shifted during disease conditions. To be able to study regulatory mechanisms and to unravel the reasons behind such a shift in mRNA/protein ratios, it is crucial to isolate mRNA and protein from the same sample.

Further, microRNAs (miRNA) regulate gene expression and dysregulation of miRNA have been implicated in a number of diseases or conditions. If microRNA can be isolated from the same sample, together with total protein, genomic DNA and total RNA, there is a clear advantage to our understanding of the interaction and effects among them. An effective means for the isolation of microRNA would also aid the development of microRNA-based diagnostics and therapeutics, in the fields of cancer, neurology, cardiology, among others.

A novel and advantageous method for carrying out isolation of genomic DNA, RNA and proteins from the same sample is presented herein.

### Summary of the Invention

The instant invention provides an improved method for rapid separation and isolation of double-stranded and single-stranded nucleic acids from the same sample. The double-stranded nucleic acid is selectively adsorbed to a mineral support in the presence of high concentration of chaotropic salt. The flowthrough containing single-stranded nucleic acid is adjusted so that single-stranded nucleic acid is adsorbed to a second mineral support. While proteins can be purified from the flow-though of the second mineral support, the nucleic acids are eluted from each of the mineral supports respectively.

Thus, one aspect of the invention provides a method for the separation and/or purification of at least two cellular components selected from genomic DNA, RNA and proteins, which method comprising:
a) generating an aqueous solution containing said cellular components by lysing a biological sample with a lysis solution;
b) applying said aqueous solution to a first mineral support under conditions such that genomic DNA binds to the first mineral support;
c) collecting the flowthrough which contains unbound RNA and proteins;
d) mixing said flowthrough from step (c) with a dipolar aprotic solvent selected from Acetone, Acetonitrile, Tetrahydrofuran (THF), Methyl Ethyl Ketone, N,N-Dimethylformamide (DMF), and Dimethyl Sulfoxide to form a mixture, then applying said mixture to a second mineral support under conditions such that RNA binds to said second mineral support; and
e) collecting the flowthrough which contains proteins.

In certain embodiments of the invention, the sample is lysed using a lysis solution containing a chaotropic salt, a non-ionic detergent and a reducing agent. Preferably, the chaotropic salt is Guanidine Hydrochloride (GuHCl). Also preferably, the non-ionic detergent is NP-40 and the reducing agent is β-Mercaptoethanol (β-ME). In other embodiments of the invention, the flowthrough from the first mineral support is admixed with an organic material prior to binding of RNA to the second mineral support. In a preferred embodiment, the organic material is a polar or dipolar aprotic solvent. Most preferably, the organic material is Acetone.

In certain embodiments, the method further comprises washing the first mineral support and eluting the genomic DNA thereof. In other embodiments, the method also includes washing the second mineral support and eluting the RNA thereof. In still other embodiments, the method also includes further isolating the protein from the flowthrough.

In another aspect, the invention provides a kit for separating and isolating double stranded nucleic acid, single stranded nucleic acid and proteins. The kit includes a lysis solution for lysing the biological sample; a first mineral support for binding the double stranded nucleic acid; a second mineral support for binding the single stranded nucleic acid; an elution solution for eluting the double stranded nucleic acid from the first mineral support; and an elution solution for eluting single stranded nucleic acid from the second mineral support. Optionally, the kit also includes means for isolating proteins from the flowthrough after genomic DNA and RNA binds to the respective mineral supports.

In a preferred embodiment, the lysis solution includes a chaotropic salt, a non-ionic detergent and a reducing agent. In still another preferred embodiment, the first mineral support and the second mineral support are each silica membranes.

The above and further features and advantages of the instant invention will become clearer from the following detailed description and claims.

### Brief Description of the Drawings

Figure 1 presents a schematic diagram of the method for the isolation of genomic DNA, total RNA and protein from a single sample, according to an embodiment of the invention.
Figure 2 shows a gel image of isolated genomic DNA and total RNA according to one embodiment of the invention.
Figure 3 shows gel images of genomic DNA and RNA samples isolated according to certain embodiments of the invention, as compared to those obtained from commercial products. Top: total RNA; bottom: genomic DNA. Left side panels show nucleic acid samples isolated from cultured HeLa cells. Right side panels show nucleic acid samples isolated from rat liver tissue.
Figure 4 is an image obtained from the Agilent Bioanalyzer of total RNA samples isolated from HeLa cell cultures, according to an embodiment of the invention, as compared to those from commercial products.
Figure 5 shows real-time PCR amplification results obtained from the genomic DNA samples from HeLa cell cultures, with very similar amplification profiles observed among the samples, including those obtained using commercial products.
Figure 6 shows real-time RT-PCR amplification results obtained from total RNA samples from HeLa cell cultures, with very similar amplification profiles observed among the samples, including those obtained using commercial products.
Figure 7 is a Coomassie staining of an SDS-PAGE gel, which shows the total protein isolated from HeLa cell cultures, according to an embodiment of the invention, as well as that isolated from a commercial product.
Figure 8 shows results obtained from Western Blotting experiments of protein samples isolated according to an embodiment of the invention, as compared to those isolated using commercial products.
Figure 9 is an image obtained from the Agilent Bioanalyzer of total RNA samples isolated from rat liver tissue, according to an embodiment of the invention, as compared to those from commercial products.
Figure 10 shows real-time PCR amplification results obtained from the genomic DNA samples from rat liver tissue, with very similar amplification profiles observed among the samples, including from commercial products.
Figure 11 shows real-time RT-PCR amplification results obtained from total RNA samples from rat liver tissue, with very similar amplification profiles observed among the samples, including those obtained using commercial products.
Figure 12 is a Coomassie staining of an SDS-PAGE gel, which shows the total protein isolated from rat liver tissue, according to certain embodiments of the invention.
Figure 13 is a Coomassie staining of an SDS-PAGE gel, which shows the total protein isolated from rat liver tissue, according to an embodiment of the invention, as well as that isolated from commercial products.
Figure 14 shows gel images and yield results of genomic DNA and total RNA isolated from HeLa cells, according to certain embodiments of the invention.
Figure 15 shows gel images and yield results of genomic DNA and total RNA isolated from rat liver tissue, according to certain embodiments of the invention.
Figure 16 shows gel images and yield results of small RNA isolated from total RNA purified according to an embodiment of the invention (Lanes 1, 2, 3), and control samples (Q and Q). The total RNA source material was loaded as another control (Lane 'input').
Figure 17 presents qRT-PCR graph for four microRNA, confirming the presence of both low and high copy number microRNA in the isolated small RNA sample.
Figure 18 compares small RNA isolation from total RNA isolated according to the current method with that from two commercial products. Small RNA is shown on the bottom panel, while "large" RNA (total RNA deprived of small RNA) is on the top panel.

### Detailed Description of the Invention

The present invention provides a method for highly effective, simple extraction of genomic DNA, RNA and proteins from a single biological material, such as cells, tissues and biological fluids. Advantageously, the method can be achieved without the use of toxic or corrosive reagents and without the use of expensive ultracentrifugation equipment. Genomic DNA and total RNA can be isolated utilizing the method of the invention in as little as 30 minutes, and proteins in as little as 45 minutes. These results are substantially faster than existing methods for the isolation of individual components.

The invention is also applicable to the separate isolation of RNA, DNA, proteins or any combination of at least two of these cellular components. The resulting genomic DNA and total RNA isolated are of high quality suitable for use in downstream applications. We have also found that compared to total RNA isolated from other commercial protocols, total RNA isolated by the current method contains a much higher level of small RNA. Thus the invention also provides a method for isolating small RNA, by subjecting the total RNA isolated according to the current method to any one of the known small RNA isolation procedures. Small RNA could therefore be isolated from the same starting sample, together with the other components (i.e., genomic DNA, total RNA and protein).

The term "biological material" or "biological sample" is used in a broad sense and is intended to include a variety of biological sources that contain nucleic acids and proteins. Such sources include, without limitation, whole tissues, including biopsy materials and aspirates; in *vitro* cultured cells, including primary and secondary cells, transformed cell lines, and tissue and blood cells; and body fluids such as urine, sputum, semen, secretions, eye washes and aspirates, lung washes and aspirates. Fungal and plant tissues, such as leaves, roots, stems, and caps, are also within the scope of the present invention. Microorganisms and viruses that may be present on or in a biological sample are within the scope of the invention. Bacterial cells are also within the scope of the invention.

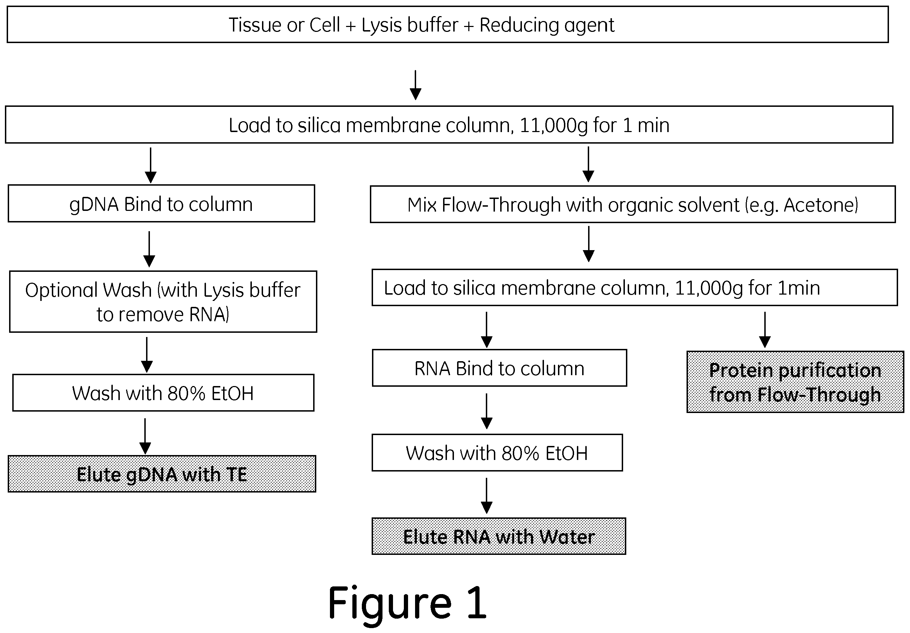

In its broadest aspects, the invention encompasses methods for isolating substantially pure and undegraded total RNA, genomic DNA and proteins from biological materials, including tissue, cells and body fluids. Accordingly, a biological sample is first lysed to generate an aqueous solution containing cellular components; then the aqueous solution is applied to a first mineral support under conditions for genomic DNA to bind; while the flowthrough containing unbound total RNA and proteins is collected. The flowthrough is applied to a second mineral support under conditions for RNA to bind; and the flowthrough thereof is collected which contains proteins. The genomic DNA and total RNA are eluted from the first and second mineral support, respectively. An example of a workflow according to one embodiment of the invention is presented in Figure 1.

Preferably, the biological sample or cells are first lysed in an aqueous lysis system containing chaotropic substances and/or other salts by, in the simplest case, adding it to the cells. The term "chaotrope" or "chaotropic salt," as used herein, refers to a substance that causes disorder in a protein or nucleic acid by, for example, but not limited to, altering the secondary, tertiary, or quaternary structure of a protein or a nucleic acid while leaving the primary structure intact. Exemplary chaotropes include, but are not limited to, Guanidine Hydrochloride, Guanidinium Thiocyanate, Sodium Thiocyanate, Sodium Iodide, Sodium Perchlorate, and Urea. A typical anionic chaotropic series, shown in order of decreasing chaotropic strength, includes: CCl₃COO⁻→CNS⁻→CF₃COO⁻→ClO₄⁻>I⁻→ CH₃COO⁻→Br⁻, Cl⁻, or CHO₂⁻.

Some of the starting materials mentioned cannot be lysed directly in aqueous systems containing chaotropic substances, such as bacteria, for instance, due to the condition of their cell walls. Therefore, these starting materials must be pretreated, for example, with lytic enzymes, prior to being used in the process according to the invention.

One of the most important aspects in the isolation of RNA and proteins is to prevent their degradation during the isolation procedure. Therefore, the current reagents for lysing the biological samples are preferably solutions containing large amounts of chaotropic ions. This lysis buffer immediately inactivates virtually all enzymes, preventing the enzymatic degradation of RNA and proteins. The lysis solution contains chaotropic substances in concentrations of from 0.1 to 10 M, such as from 1 to 10 M. As said chaotropic substances, there may be used, in particular, salts, such as Sodium Perchlorate, Guanidinium Chloride, Guanidinium Isothiocyanate/Guanidinium Thiocyanate, Sodium Iodide, Potassium Iodide, and/or combinations thereof.

Preferably, the lysis solution also includes a reducing agent which facilitates denaturization of RNase by the chaotropes and aids in the isolation of undegraded RNA. Preferably, the reducing agent is 2-Aminoethanethiol, tris-Carboxyethylphosphine (TCEP), or β-Mercaptoethanol.

Optionally, the lysis solution also includes a non-ionic surfactant (i.e., detergent). The presence of the detergent enables selective binding of genomic DNA to the mineral support. Exemplary nonionic surfactants include, but are not limited to, t-Octylphenoxypolyethoxyethanol (TRITON X-100™), (octylphenoxy)Polyethoxyethanol (IGEPAL™ CA-630/NP-40), Triethyleneglycol Monolauryl Ether (BRIJ™ 30), Sorbitari Monolaurate (SPAN™ 20), or the Polysorbate family of chemicals, such as Polysorbate 20 (i.e., TWEEN™ 20). Other commercially available Polysorbates include TWEEN™ 40, TWEEN™ 60 and TWEEN™ 80 (Sigma-Aldrich, St. Louis, MO). Any of these and other related chemicals is effective as a replacement of TWEEN™ 20.

An effective amount of non-ionic detergent for selective binding of double-stranded nucleic acid could vary slightly among the different detergents. However, the optimal concentration for each detergent (or combination of detergents) can be easily identified by some simple experiments. In general, it is discovered that a final concentration of detergent at 0.5% or greater is effective for selective binding of the double-stranded nucleic acid. In certain embodiments, the effective concentration is between 0.5% and about 10%. In a preferred embodiment, the concentration is between 1% and 8%. It is also noted that more than one non-ionic detergent can be combined, as long as the combined concentration of the detergents is within the range of 0.5% to about 10%.

It is discovered that at certain concentrations, the presence of the detergents not only improves nucleic acids recovery but also reduces double-stranded nucleic acid contamination in single-stranded nucleic acids purified. This is at least partly achieved through improved binding of genomic DNA on silica membrane.

In a preferred embodiment, the lysis solution includes NP-40 (IGEPAL™ CA-630). In a most preferred embodiment, the lysis solution includes Guanidine HCl, TWEEN™ 20, NP-40 and β-Mercaptoethanol.

The lysis solution of the present invention preferably also contains a sufficient amount of buffer to maintain the pH of the solution. For the simultaneous isolation of RNA, DNA and proteins, the pH should be maintained in the range of about 5-8. The preferred buffers for use in the lysis solution include tris-(hydroxymethyl)Aminomethane Hydrochloride (Tris-HCl), Sodium Phosphate, Sodium Acetate, Sodium Tetraborate-boric Acid and Glycine-sodium Hydroxide.

Once the biological samples are lysed, the aqueous solution containing cellular components is applied to a mineral support. It is discovered that under conditions of the lysis solution (with certain amounts of non-ionic detergent), virtually all the genomic DNA binds to the first mineral support, while the total RNA and proteins do not bind and are collected as the flowthrough by a simple spin.

The mineral support preferably consists of porous or non-porous metal oxides or mixed metal oxides, silica gel, silica membrane, materials predominantly consisting of glass, such as unmodified glass particles, powdered glass, Quartz, Alumina, Zeolites, Titanium Dioxide, Zirconium Dioxide. The particle size of the mineral support material ranges from 0.1 µm to 1000 µm, and the pore size from 2 to 1000 µm. Said porous or non-porous support material may be present in the form of loose packings or may be embodied in the form of filter layers made of glass, quartz or ceramics, and/or a membrane in which silica gel is arranged, and/or particles or fibers made of mineral supports and fabrics of quartz or glass wool, as well as latex particles with or without functional groups, or frit materials made of Polyethylene, Polypropylene, Polyvinylidene Fluoride, especially ultra high molecular weight polyethylene, high density polyethylene.

The flowthrough from the first mineral support contains total RNA and proteins. The flowthrough is mixed with an organic solvent and applied to a second mineral support. It is discovered that at the presence of certain organic solvents, RNA binds to the mineral support while the proteins do not. A simple centrifugation step separates the RNA bound to the mineral support from the proteins in the flowthrough.

As an example, polar protic solvents such as lower aliphatic alcohol are suitable organic solvents. Preferably the organic solvents are dipolar aprotic solvents. Suitable dipolar aprotic solvents include but are not limited to Acetone, Tetrahydrofuran (THF), Methyl ethyl Ketone, Acetonitrile, N,N-Dimethylformamide (DMF), and Dimethyl Sulfoxide (DMSO). Most preferably, the organic solvent is Acetone or Acetonitrile.

The second mineral support for RNA binding consists of a similar material as the first mineral support described above. Preferably, the first mineral support and the second mineral support are each silica membranes.

It is envisioned that in certain applications, it would be advantageous to allow both genomic DNA and RNA to bind together to the same mineral column. This can be achieved by the addition of the organic solvent such as dipolar aprotic solvent prior to the loading of the first column. Separation of the DNA and RNA can be realized by conventional techniques such as by controlling elution condition. Alternatively, one of the nucleic acids can be removed by enzymatic reaction.

After optionally performed washing steps, the double-stranded nucleic acid adsorbed on the first mineral support and the single-stranded nucleic acid adsorbed on the second mineral support can be eluted under conditions of low ionic strength or with water, respectively.

Optionally, washing steps may also be performed prior to the elution of the respective nucleic acid (single-stranded nucleic acid or double-stranded nucleic acid). For purifying the double-stranded genomic DNA, an optional wash of the first mineral support (i.e., column) with the lysis buffer removes any residual amount of RNA. Further, a washing buffer containing a high concentration of organic solvents such as lower aliphatic alcohols, can be used for both genomic DNA and RNA purification, to remove components other than the desired nucleic acids by a quick centrifugation step.

Following the workflow illustrated in Figure 1 and the experimental conditions as further described in the Examples 2 and 3 below, DNA and total RNA have been successfully purified from biological samples. Table 1 presents DNA and total RNA isolation data obtained using cultured cells as well as rat liver tissues. Good quality nucleic acids are obtained from these experiments by comparison with current industry standards or other commercially available purification kits (see below).

**Table 1: DNA and total RNA isolation from 9 samples each.**

| **RNA** | **1x10^6 HeLa Cells** | **Typical output or result** |
|---|---|---|
| | Yield (µg) | **10** |
| | Purity (A₂₆₀/A₂₈₀) | **2.10** |
| | 28s:18s | **2** |
| | RIN | **9.8** |
| | gDNA contamination | - |
| **DNA** | **1x10^6 HeLa Cells** | |
| | Yield (µg) | **5** |
| | Purity (A₂₆₀/A₂₈₀) | **1.87** |
| | Size (Kb) | **30** |
| | RNA contamination | - |
| **RNA** | **10 mg Rat Liver** | |
| | Yield (µg) | **39** |
| | Purity (A₂₆₀/A₂₈₀) | **1.98** |
| | 28s:18s | **1.5** |
| | RIN | **8.7** |
| | gDNA contamination | **<3%** |
| **DNA** | **10 mg Rat Liver** | |
| | Yield (µg) | **9** |
| | Purity (A₂₆₀/A₂₈₀) | **1.89** |
| | Size (Kb) | **20-25** |
| | gDNA degradation | - |
| | RNA contamination | - |

The proteins in the flowthrough can be further purified with per se known methods, such as precipitation, gel filtration or hydrophobic interaction chromatography (HIC). Preferably, the proteins are purified by precipitation. More preferably, the proteins are purified by precipitation at the presence of a divalent metal cation. Most preferably, the proteins are isolated by precipitation at the presence of ZnSO₄. Table 2 shows the protein yield obtainable using different downstream protein isolation methods.

**Table 2: Protein yield using different downstream protein isolation protocols.**

| Method | Total Number of Experiments | Mean (µg) | StDev |
|---|---|---|---|
| 2-D Clean-Up Kit (GE Healthcare) | 9 | 936.7 | 148.5 |
| TCA | 9 | 787.3 | 77.1 |
| ZnSO₄ | 9 | 786.3 | 198.8 |

The invention also provides a method for isolating small RNA from the same sample. Briefly, total RNA purified by the above method contains a much higher level of small RNA compared to total RNA isolated from other commercial protocols, thus enabling the isolation of small RNA, including microRNA, from the purified total RNA. We show that commercial microRNA isolation kits are effective in isolating small RNA from total RNA sample acquired by the current method. The isolated small RNA includes microRNA of both high copy number as well as low copy number.

A kit may be used for the separation and/or purification of genomic DNA, total RNA and proteins from a biological sample. The kit comprises: a lysis solution for lysing the biological sample; a first mineral support for binding the genomic DNA; a second mineral support for binding the RNA; an elution solution for eluting genomic DNA from the first mineral support; an elution solution for eluting RNA from the second mineral support, and an organic solvent such as Acetone. Optionally, the kit also includes means for isolating proteins from the flowthrough after genomic DNA and RNA binds to the respective mineral supports, as well as a user manual.

Preferably, the lysis solution in the kit includes a chaotropic salt, a non-ionic detergent and a reducing agent. Most preferably, the lysis solution includes Guanidine HCl, TWEEN™ 20, NP-40 and β-Mercaptoethanol.

The mineral support may be present in loose packing, fixed between two means, or in the form of membranes which are arranged within the hollow body of a column. Preferably, the first mineral support and the second mineral support are each silica membranes.

Other features and advantages of the invention will be apparent from the following examples and from the claims.

### Examples

The following examples serve to illustrate the process for the isolation of genomic DNA, RNA and protein from a single source according to embodiments of the present invention and are not intended to be limiting.

### Solutions and protocols

### 1. Solutions and Columns Used in the Examples

| **Description** | **Composition** |
|---|---|
| Lysis buffer | 7 M Guanidine HCl, 50mM Tris, 2% TWEEN™ 20, pH 7 (β-Mercaptoethanol added to 1%) |
| Wash buffer | 10mM Tris, 1mM EDTA, pH 8 (before use, 4 parts of Ethanol added to 1 part of buffer) |
| Genomic DNA Elution buffer | 10mM Tris, 0.5mM EDTA, pH 8 |
| RNA Elution buffer | Water |
| ILLUSTRA genomicPrep tissue and cell mini column | Silica membrane spin column |

### 2. Sample Disruption and Homogenization

### 2.1 Cells Disruption and Homogenization:

a. Pellet 1 x 10⁶ cultured cells in a 1.5 ml microcentrifuge tube by centrifugation at 8000x g for 1 minute.
b. Completely remove the supernatant by aspiration.
c. Add 350 µl of Lysis buffer (containing ß-ME).
d. Vortex to resuspend the cell pellet.
e. Homogenize the lysate by passing it through a 20-gauge needle fitted to an RNase and DNase-free syringe for at least 5 times.
f. Proceed to step 3.

### 2.2 Tissue Disruption and Homogenization using the POLYTRON™ Homogenizer (Kinematica AG, Switzerland):

a. Place 10 mg tissue in a suitable sized tube.
b. Add 350 µl of Lysis buffer (containing βME).
c. Homogenize the tissue according to the POLYTRON™'s user manual.
d. Visually inspect the prepared homogenate and ensure thorough homogenization.
e. Proceed to step 3.

### 2.3 Tissue Disruption using a mortar and pestle followed by homogenization using needle and syringe:

a. Immediately place the weighed tissue in liquid nitrogen, and grind thoroughly with a mortar and pestle.
b. Decant tissue powder and liquid nitrogen into an RNase-free, liquid-nitrogen-cooled, 2 ml microcentrifuge tube.
c. Allow the liquid nitrogen to evaporate, but do not allow the tissue to thaw.
d. Add the appropriate volume of Lysis buffer, and homogenize by passing lysate at least 5 times through a 20-gauge needle fitted to an RNase-free syringe.

### 3. gDNA purification

### 3.1 gDNA Binding

a. Place a new spin column into a new collection tube.
b. Transfer the homogenized lysate from step 2 (∼ 350 µl) to the column.
c. Centrifuge at 11 000x g for 1 min.
d. Save the flowthrough for purification of RNA and Protein.
e. Transfer the column to a new 2 ml collection tube.

### 3.2 Column Wash

a. Add 500 µl of Lysis buffer to the column.
b. Centrifuge at 11 000x g for 1 min. Discard the flowthrough.
c. Place the column back into the same collection tube.
d. Add 500 µl of Wash buffer to the column.
e. Centrifuge at 11 000x g for 1 min.
f. Transfer the column to a DNase-free 1.5 ml microcentrifuge tube.

### 3.3 gDNA Elution

a. Add 100 µl of gDNA Elution buffer to the center of the column.
b. Centrifuge at 8 000x g for 1 minute.
c. Discard the column and store the tube containing pure gDNA at -20°C.

### 4. Total RNA purification

### 4.1 RNA Binding

a. Place a new spin column in a new collection tube.
b. Add 350 µl of 100% Acetone to the flowthrough from step 3.1.d. Mix well by pipetting up and down several times. Transfer the entire mixture to the column.
c. Centrifuge at 11 000x g for 1 min.
d. Save the flowthrough for protein purification.
e. Transfer the column to a new 2 ml collection tube.

### 4.2 Column Wash

a. Add 500 µl of Wash Buffer to the column.
b. Centrifuge at 11 000x g for 1 min.
c. Transfer the column to an RNase free 1.5 ml microcentrifuge tube.

### 4.3 RNA Elution

a. Add 100 µl of Elution buffer to the center of the column.
b. Centrifuge at 8000x g for 1 minute.
c. Discard the column and store the tube containing pure RNA at -80°C until needed.

### 5. Total Protein Purification Using 2-D Clean-Up Kit (GE Healthcare)

**NOTE:** All steps should be carried out on ice unless otherwise specified.

### 5.1 Protein Precipitation

a. Use the flowthrough from step 4.1 as starting point
   for protein precipitation. Mix well and transfer 100 µl of flowthrough to a new
   1.5 ml microcentrifuge tube.
b. Add 300 µl of Precipitant and mix well. Incubate on ice for 15 min
c. Add 300 µl of Co-Precipitant to the mixture. Mix briefly.
d. Centrifuge tubes at maximum speed for 5 minutes.
e. Remove the supernatant by pipetting or decanting as completely as possible
f. Add 40 µl of Co-Precipitant on top of the pellet and incubate on ice for 5 minutes.
g. Centrifuge the tubes at maximum speed for 5 minutes. Carefully remove and discard the supernatant.

### 5.2 Protein Pellet Wash

a. Pipet 25 µl of de-ionized water to the pellet. Vortex the tube for 5 minutes.
b. Add 1ml of pre-chilled Wash Buffer and 5 µl of Wash Additive to each tube. Vortex vigorously. (Note: pellet will not dissolve in wash buffer.)
c. Incubate tube at -20°C for 30 minutes, vortex 20-30 s once every 10 minutes.
d. Centrifuge tubes at maximum speed for 5 minutes.
e. Carefully remove and discard the supernatant. A white pellet should be visible at this step.
f. Keeping the lid open, dry the precipitate for a maximum of 5 minutes at room temperature.

### 5.3 Protein Pellet Resuspension

a. Add up to 100 µl of 5% SDS or 7 M Urea and mix vigorously to dissolve the protein pellet. Use the tip of the pipette to break up the pellet.
b. Incubate for 3 minutes at 95 °C to completely dissolve and denature the protein. Then cool the sample to room temperature.
c. Centrifuge at 11 000x g for 1 minute to pellet any residual insoluble material. Use supernatant in downstream applications, i.e. SDS-PAGE and Western Blotting.

Samples can be stored at -20°C for several months or at 4°C for several days.

### 6. Total Protein Isolation (Precipitation using ZnSO₄)

### 6.1 Protein Precipitation

a. Use the flowthrough from step 4.1.d as starting point for protein precipitation.
b. Add 600 µl of 10% ZnSO₄ solution.
c. Mix vigorously and incubate at room temperature for 10 minutes to precipitate the proteins.
d. Centrifuge for 10 minutes at 16 000x g.
e. Carefully remove supernatant by pipetting or decanting.

### 6.2 Wash protein pellet

a. Add 500 µl of 50% Ethanol to protein pellet.
b. Centrifuge for 1 minute at 16 000x g.
c. Remove the supernatant by using a pipet or by decanting as much liquid as possible.
d. Keep lid open and dry precipitate for 5-10 minutes at room temperature.

### 6.3 Protein Pellet Resuspension

a. For protein quantification prior to SDS-PAGE add minimum of 100 µl of 5% SDS (or 7 M Urea) and mix vigorously to dissolve the protein pellet. Use the tip of the pipet to break up the pellet.
b. If needed, add up to 1 ml of 5% SDS (or 7 M Urea) to completely dissolve the pellet.
c. Incubate for 5 minutes at 95°C to completely dissolve and denature the protein. Vortex vigorously.
d. Allow the sample to cool to room temperature for 5 minutes.
e. Centrifuge for 1 minute at 16 000x g.
f. Transfer the supernatant to a new 1.5 ml microcentrifuge tube.
g. For SDS-PAGE and Western Blotting use the supernatant.
h. Store the samples at -20°C for several months or at 4°C for several days.

### Example 1: Optimization of the workflow

In an effort to find an optimal workflow, we tested a variety of solutions and additives for the extraction and purification of genomic DNA, total RNA and total proteins from HeLa cell cultures.

We found that a lysis buffer containing a mixture of 7 M Guanidine HCl, 50mM Tris-HCl, pH 7 (with or without detergent, e.g. 5% NP-40 or TWEEN™ 20) works well in the presence of a reducing agent (e.g., TCEP or β-ME). Alternatively, a lysis solution containing a mixture of 7 M Guanidine HCl, 50mM Tris-HCl, pH 5 (with detergent, e.g. 5% NP-40) also works well in the presence of a reducing agent (e.g., TCEP or β-ME). The biological sample, when mixed with either solution, was homogenized according to the protocol provided above and loaded onto a silica membrane column. A quick spin removed the mixture as flowthrough and the genomic DNA bound to the column. The column containing the genomic DNA was further processed according to the protocol above to isolate pure genomic DNA.

The flowthrough contains total RNA as well as proteins. For the further separation of total RNA from proteins, 0.7 volume of Acetone was found to be effective for selectively attaching the total RNA to a silica membrane column. Thus 0.7 volume of Acetone was added to the flowthrough, then the mixture was loaded to a silica membrane column. A quick spin separated the mixture as flowthrough which now contains the protein and the column with RNA bound thereon. The column was further processed according to the protocol above to isolate pure RNA, while the flowthrough was used for protein purification.

We have also tested polar protic solvents such as lower aliphatic alcohols, as well as dipolar aprotic solvents. As expected, lower aliphatic alcohols enable RNA binding to the silica membrane column. We found that a number of dipolar aprotic solvents are useful for this purpose as well. In particular, Acetone and Acetonitrile were found to be more preferable than the others, although other dipolar aprotic solvents tested were found to work as well (data not shown).

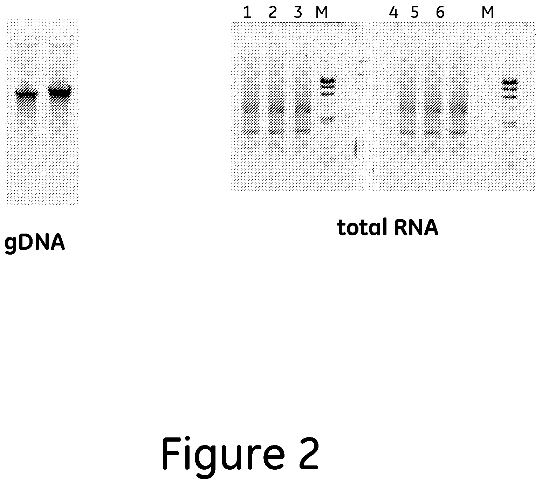

Figure 2 shows gel images of genomic DNA and total RNA isolated from this process. The starting material was 1 million HeLa cells. The lysis solution contained 7 M GuHCl, 50mM Tris-HCl, pH 7, 5% TWEEN™ 20 and 1% TCEP. Prior to loading of the second mineral support, the flowthrough was mixed with Acetone or Acetonitrile. The protocols above were followed otherwise. Genomic DNA was eluted in a final volume of 200 µl. Total RNA was eluted in a final volume of 100 µl. Each lane of the gel contained 10 µl eluted sample. It is clear that the protocol worked well for the isolation and purification of both genomic DNA and total RNA (lanes 1-3: total RNA isolated using Acetone; 4-5: total RNA isolated using Acetonitrile; M: Lambda HindIII marker).

### Example 2: Isolation of Genomic DNA, RNA and protein from cultured cells

Cultured cells were further tested for the performance of the workflow. We also tested the workflow in comparison with commercial products, i.e., the AllPrep kit (Qiagen Inc., Valencia, CA) and the NUCLEOSPIN™ RNA/Protein kit (plus DNA elution buffer set, MACHEREY-NAGEL GmbH & Co. KG, Germany). Multiple samples were processed to assess the consistency of the protocol. The purity of the products was assessed by UV spectrophotometry and by gel analysis. The samples obtained were also evaluated in downstream applications such as real-time PCR, RT-PCR, and Western Blotting experiments. Our results show that the protocol as described above works consistently and well for cultured cells.

We started from 1x10⁷ HeLa cell culture. The cells were pelleted and diluted to 1x10⁶ aliquots prior to the start of the preparation. Each of three operators followed the same protocol above or from the manufacturers for one of the aliquots. The optional steps in each of the protocols were not performed. Genomic DNA and RNA were isolated on the first day. The protein flowthrough was further purified on the following day, with three different methods: 2-D Clean-Up Kit (GE Healthcare, Piscataway, NJ), NUCLEOSPIN™ Protein Pecipitator kit (Macherey-Nagel) and AllPrep Protein Precipitation kit (Qiagen).

The genomic DNA and total RNA isolation yield results are shown in Table 1 above. The protein purification yield results are shown in Table 2 above. It can be seen that the protocol produces consistent, high quality results in isolating genomic DNA, total RNA and proteins.

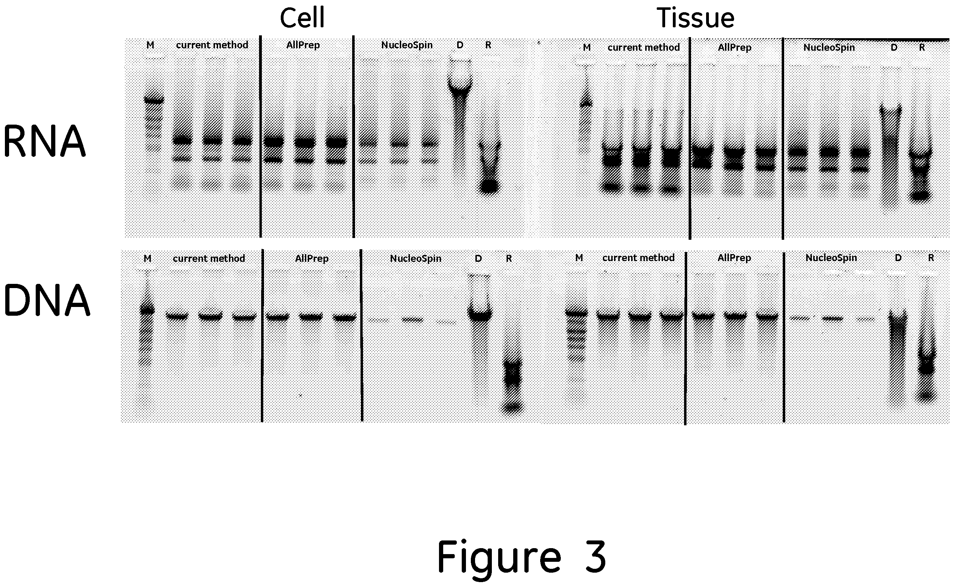

The purity of the genomic DNA and RNA was also examined by agarose gel analysis. Figure 3 shows gel images of genomic DNA and RNA samples isolated, as compared to commercial products. Top: total RNA; bottom: genomic DNA. Left side panels show nucleic acid samples isolated from cultured HeLa cells. Right side panels show nucleic acid samples isolated from rat liver tissue (see Example 3). M: Marker lambda/Hind III (100 ng); D: rat genomic DNA control (400 ng); R: rat liver total RNA control (600 ng). For genomic DNA, 2 µl was loaded per well for current method and NUCLEOSPIN™, while 4 µl was loaded for AllPrep. For total RNA, 5 µl was loaded per well for current method and AllPrep, while 3 µl was loaded for NUCLEOSPIN™. It is clear from the gel images that the genomic DNA and RNA isolated are pure and with little cross contamination.

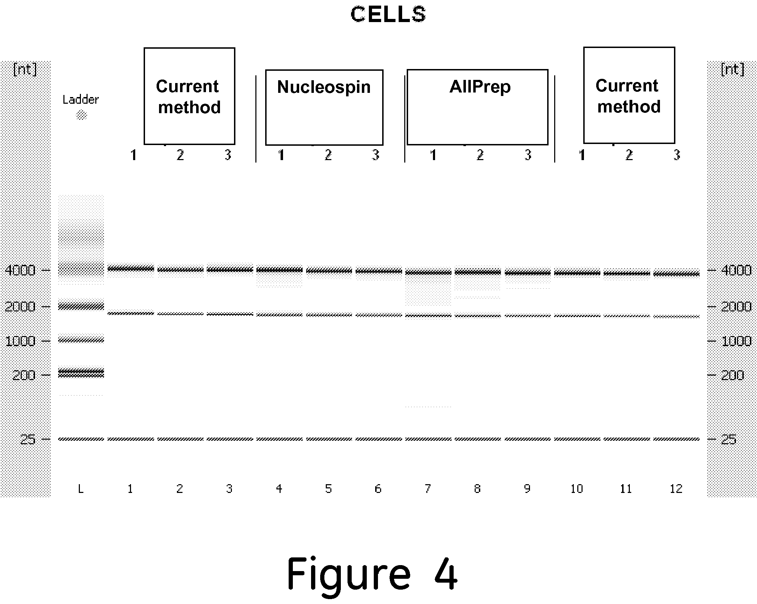

We also analyzed total RNA isolated using the Agilent Bioanalyzer (Agilent Technologies, Inc., Santa Clara, CA). Again, the images show similar results from the different protocols (Figure 4).

The quality of the purified genomic DNA was assessed by real-time PCR assay. Real-time PCR reactions were set up using 100 ng of purified genomic DNA per sample using the PuReTaq READY-TO-GO™ PCR beads (GE Healthcare, Piscataway, NJ) in the presence of GELSTAR™ dye (Cambrex, Baltimore, MD) using primers specific for the GAPDH gene.

### Real-time PCR reaction

Dilute genomic DNA template to 20ng/µl in water.

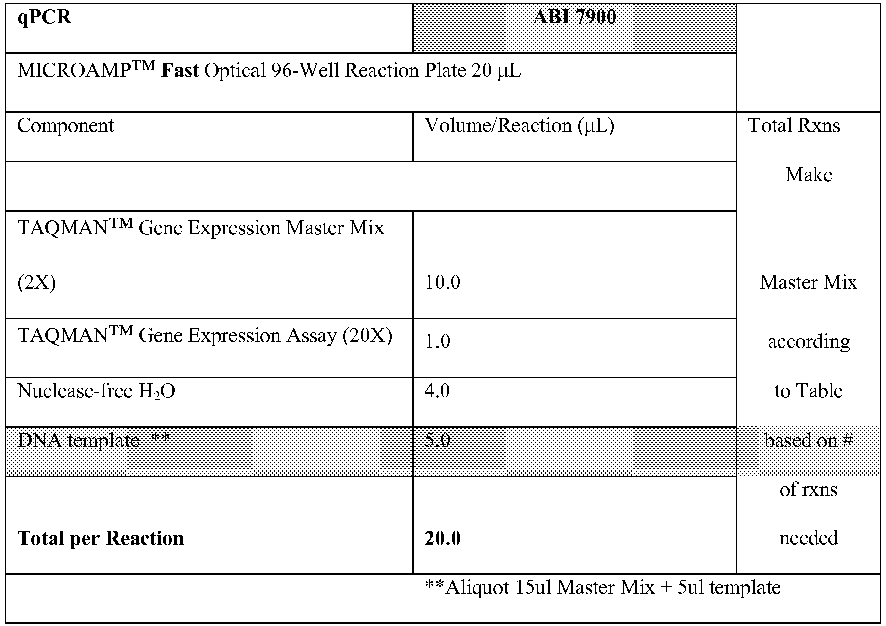

The amplification was monitored on an ABI7900HT Fast Real-time PCR System (Applied Biosystems Inc., Foster City, CA), following these cycling conditions:

| | **UDG Incubation** | **AMPLITAQ GOLD™, UP Enzyme Activation** | **PCR** | |
|---|---|---|---|---|
| **Step** | HOLD | HOLD | CYCLE (40 Cycles) | |
| | | | Denature | Anneal/ Extend |
| Time | 2 min | 10 min | 15 sec | 1 min |
| Temp | 50 °C | 95 °C | 95 °C | 60 °C |

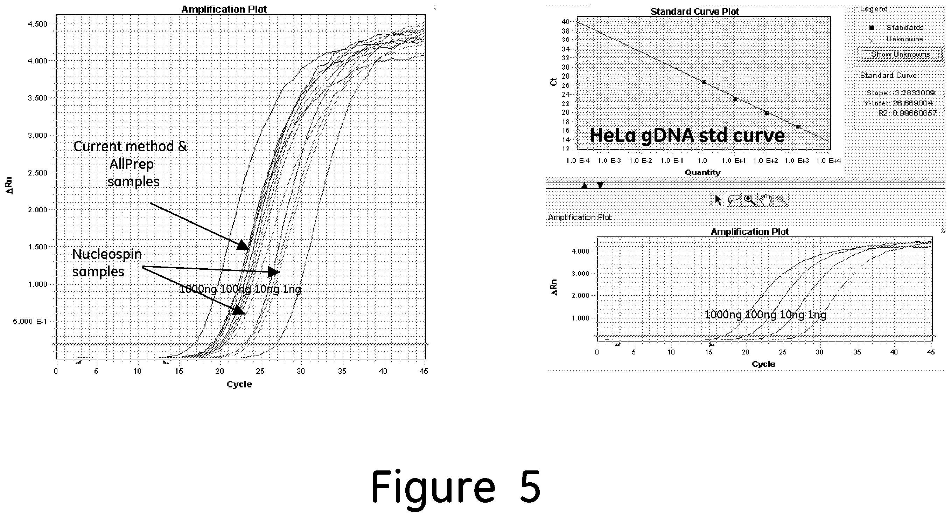

The amount of signal correlates with amplification of the GAPDH gene. The point at which signal rises above background threshold is defined as Ct value for the amplification. All the samples tested show very similar amplification profiles (Figure 5).

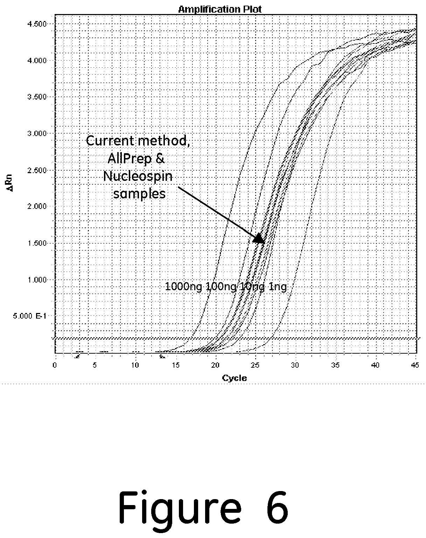

Similarly, total RNA was tested by real-time RT-PCR. Figure 6 shows amplification results obtained, with very similar amplification profiles observed among the samples, including from commercial products.

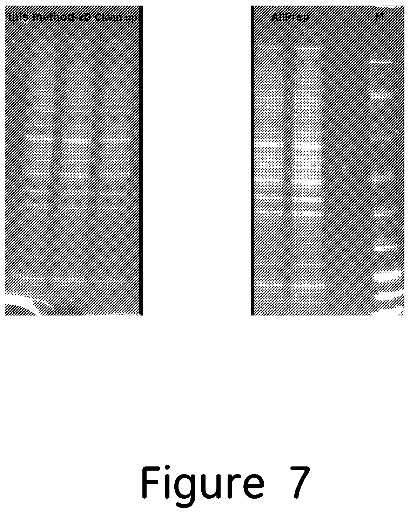

The protein isolated was analyzed on an SDS-PAGE gel, with Coomassie staining. The flowthrough from the RNA column was processed using the modified 2-D Clean-Up Kit protocol. The precipitated proteins were reconstituted with 50 µl 5% SDS. Each well was loaded with 5µl sample protein. Figure 7 shows that total proteins isolated from HeLa cell cultures, is comparable between the protocol from the current invention and that of a commercial product (AllPrep).

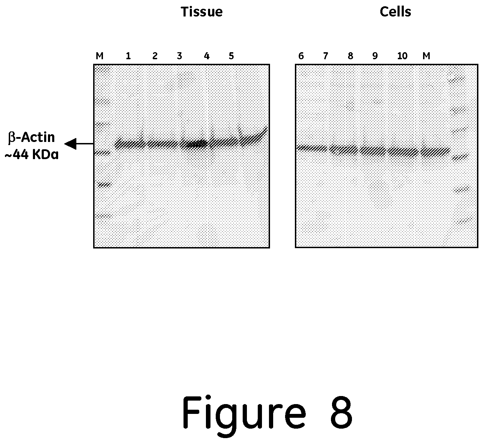

The protein samples were also analyzed using Western Blotting experiments with anti-β-actin antibody and compared to commercial products. Results are shown in Figure 8. M: Full-Range Rainbow Molecular Weight Markers (GE Healthcare). Lanes 1-2, 6-7 and 10: flowthrough from current protocol with 2-D Clean-Up Kit. Lanes 3-4 and 8-9, AllPrep Protein ppt. Lane 5, NUCLEOSPIN™ flowthrough with Macherey-Nagel Protein Precipitator. For protein isolated from HeLa cells, 5µg was used per well, for protein isolated from tissues, 10µg was used (See Example 3).

The analysis of the purified genomic DNA, RNA and proteins demonstrate clearly that the workflow works well for cultured cells.

### Example 3: Isolation of Genomic DNA, RNA and protein from tissue samples

We tested a variety of tissue sources for the performance of the workflow, including rat liver, spleen and lung. We also tested the workflow in comparison with commercial products, i.e., the AllPrep kit and the NUCLEOSPIN™ RNA/Protein kit (plus DNA elution buffer set). Multiple samples were processed to assess the consistency of the protocol. The purity of the product was measured by UV spectrophotometry and by gel analysis. The genomic DNA obtained was also evaluated in downstream applications such as real-time PCR, RT-PCR, and Western Blotting experiments. Our results show that the protocol as described above works consistently and well for tissue samples.

As an example, details are provided here for the isolation of genomic DNA, RNA and proteins from rat liver. 10 mg of rat liver tissue was homogenized using the POLYTRON™ homogenizer. The experiments are designed similarly to that of Example 2. Briefly, each of three operators followed the same protocol above or from the manufacturers to process an aliquot of the lysate. The optional steps in each of the protocols were not performed. Genomic DNA and RNA were isolated on the first day. The protein flowthrough was further purified on the following day, with different methods, including 2-D Clean-Up kit, NUCLEOSPIN™ Protein Pecipitator kit and AllPrep Protein Precipitation kit.

The genomic DNA and total RNA isolation results are shown in Table 1 above. The protein isolation results are shown in Table 2 above. It can be seen that the protocol produces consistent, high quality genomic DNA, total RNA and proteins.

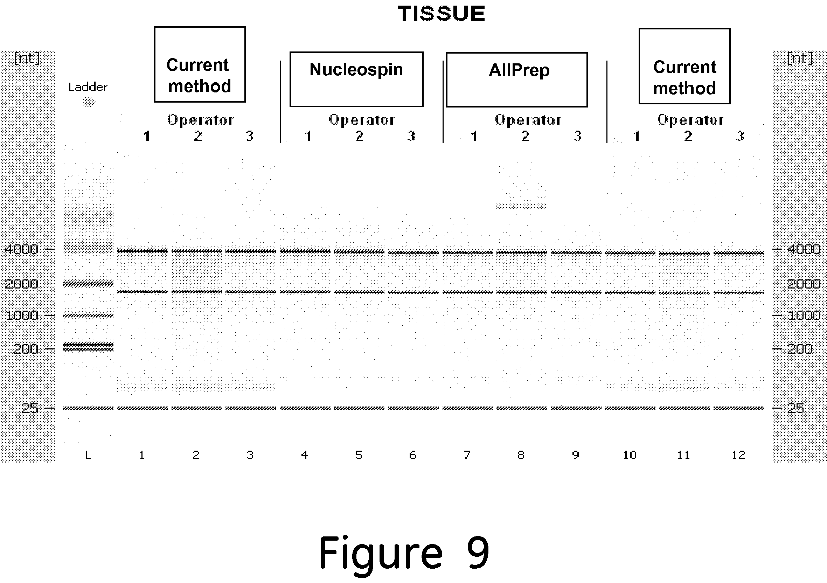

The purity of the genomic DNA and RNA was also examined by agarose gel analysis. Figure 3 shows gel images of genomic DNA and RNA samples isolated (details see Example 2). It is clear from the gel images that the genomic DNA and RNA isolated are pure and with little cross contamination. We also analyzed total RNA isolated using the Agilent Bioanalyzer. Again, the images show similar results among the different protocols (Figure 9).

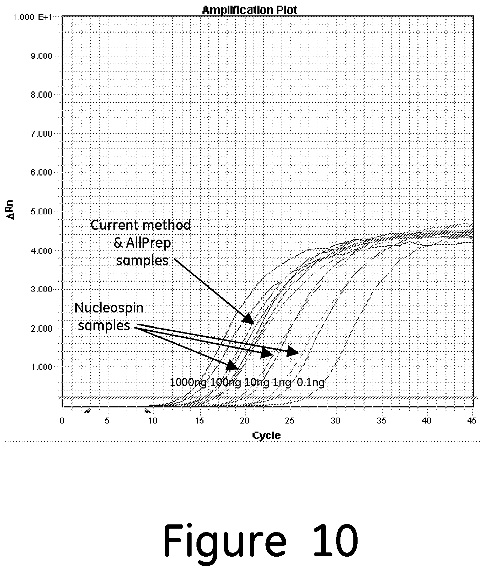

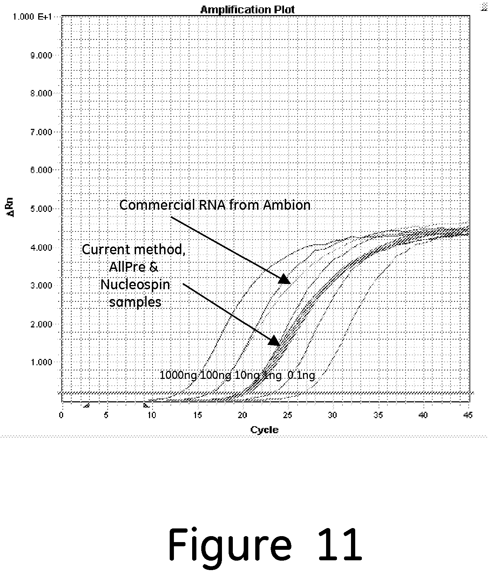

The quality of the purified genomic DNA was assessed by real-time PCR assay. Real-time reactions were set up using 100ng of purified genomic DNA per sample according to the protocol of Example 2. All the samples tested show very similar amplification profiles (Figure 10). Similarly, total RNA was tested by real-time RT-PCR. Figure 11 shows amplification results obtained, with very similar amplification profiles observed among the samples, including from commercial products.

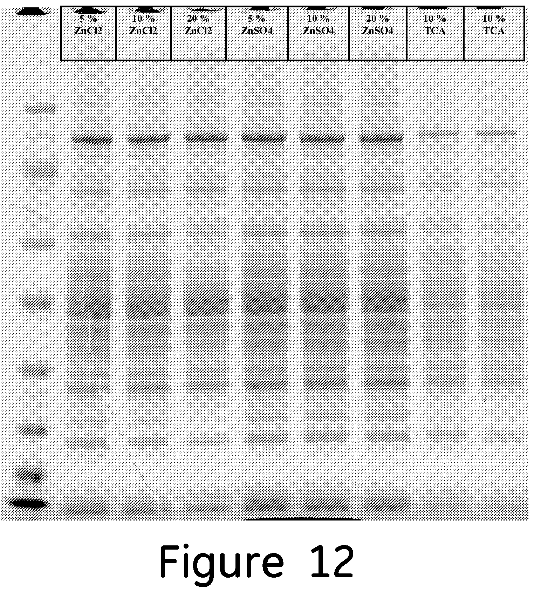

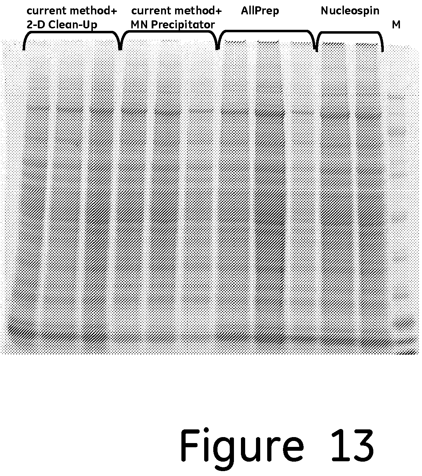

The protein isolated was analyzed on an SDS-PAGE gel, with Coomassie staining. The protein flowthrough from the current protocol was processed using a variety of methods, including precipitation using different amount of ZnSO₄ or TCA. The precipitated proteins were reconstituted with 700 µl 5% SDS. Each well was loaded with 10µl sample. Figure 12 shows that the profile of total protein isolated from rat liver is comparable among the different precipitation protocols. Figure 13 shows that the protein flowthrough from the current protocol can be further purified using the 2-D Clean-Up Kit as well as the Macherey-Nagel Protein Precipitator kit. The yield is similar to that from the commercial AllPrep kit or the NUCLEOSPIN™ kit (Figure 13).

The protein samples were also analyzed using Western Blotting experiments and compared to commercial products (Figure 8, see Example 2 for details).

The analysis of the purified genomic DNA, RNA and proteins demonstrate clearly that the workflow works well for tissue samples.

### Example 4: Increased Amount of Non-ionic Detergent Improves the Yield of Both Genomic DNA and Total RNA

As illustrated by Examples 2 and 3, our standard Lysis buffer with 2% TWEEN™ 20 works well. However, we found that an increased amount of non-ionic detergent improves binding of genomic DNA to the silica membranes on the first instance, thereby increases the recovery of both genomic DNA and total RNA. Thus, Example 1 was performed with a 5% TWEEN™ 20 in the Lysis buffer. An additional benefit with increased detergent level is a decrease of cross-contamination of genomic DNA in the total RNA isolated, due at least partly to improved binding of genomic DNA on silica membrane in the first instance.

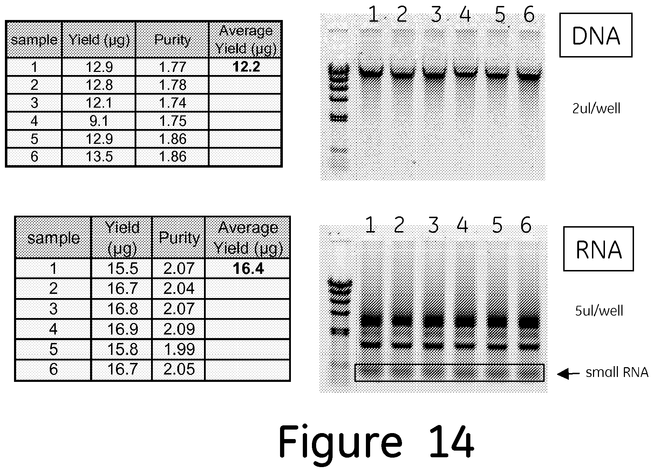

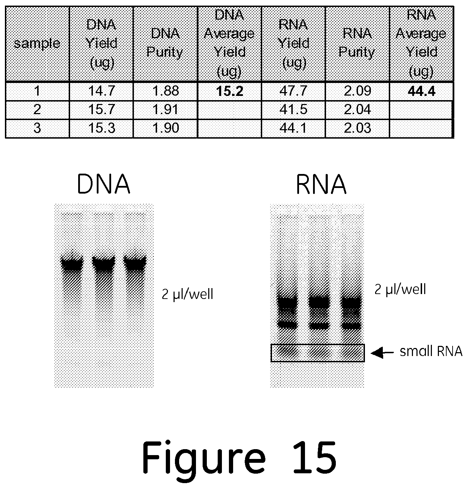

We discovered that any of a number of non-ionic detergents (e.g., TWEEN™ 20, NP-40, TRITON X-100™) could achieve this effect. Various combinations of these detergents also are effective. Further, this increased amount of detergent could be part of the Lysis solution, or it could be added just prior to binding of the sample to the silica membrane column. We present here results obtained with an optimal combination of TWEEN™ 20 and NP-40. Namely, a combination of 2% TWEEN™ 20 and 5% NP-40 was used in the lysis buffer to replace the 2% TWEEN™ 20. While other solutions and protocols were followed as stated in the beginning section of the Examples, this adjustment in detergent combination and level resulted in greatly reduced genomic DNA contamination in the total RNA isolated. Further, the yield of both genomic DNA and total RNA was also increased. The isolation of total protein was not adversely affected by this increase of detergent level in the Lysis buffer (data not shown). Figure 14 presents gel images and yield results from HeLa cell samples of 1 million cells each. Figure 15 presents those obtained from rat liver samples of 10 mg each.

### Example 5: Total RNA Isolated Contains High Levels of Small RNA

In the isolated total RNA, we observed a high concentration of small RNA molecules (See Figures 14 and 15). Here we present data showing enrichment and isolation of small RNA (less than 200 nt in length) from total RNA samples purified from the protocols above, and compare the small RNA isolated with those isolated using commercial microRNA isolation kit.

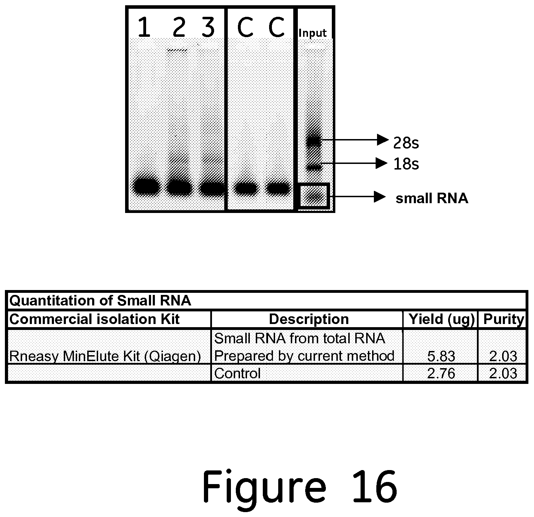

We first purified small RNA using commercial kit from Qiagen (miRNeasy Mini Kit Qiagen, Cat # 217004), from total RNA isolated according to the current invention. Briefly, 30 µg of total RNA isolated above (equivalent to about two thirds of the total RNA isolated from 10 mg of rat liver tissue) were used to purify small RNA, according to the protocol of the commercial kit. As a control, small RNA was also isolated directly from 10 mg of rat liver tissue using the protocol provided in the miRNeasy Mini Kit. Figure 16 shows the results. Lanes 1, 2, 3 are small RNA purified from total RNA isolated according to the current protocol. Lanes labeled as C show control small RNA isolated directly from rat liver tissue. We also run the total RNA isolated as another control (e.g. Lane labeled as input). It is clear that more small RNA can be isolated following the current method than directly from tissue sample.

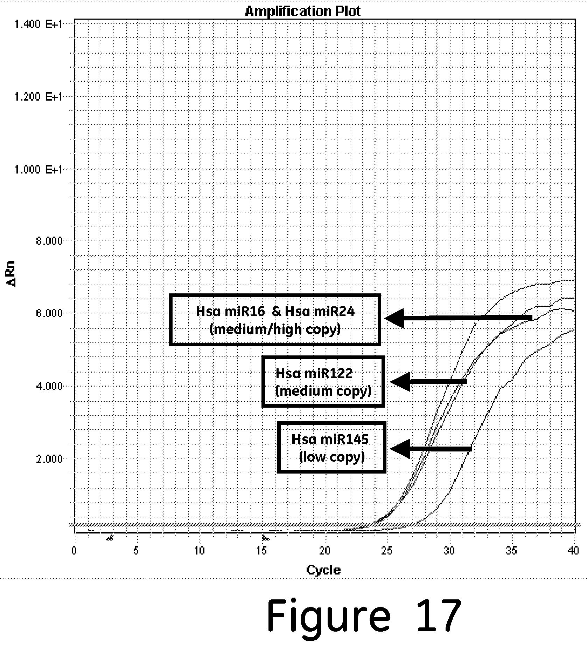

To verify that the small RNA isolated contains microRNA, we performed qRT-PCR assay using four different microRNAs of varying copy numbers. We were successful in detecting all four microRNAs in the sample (Figure 17). Thus we have successfully isolated microRNA using commercially available kit from total RNA purified from the current method.

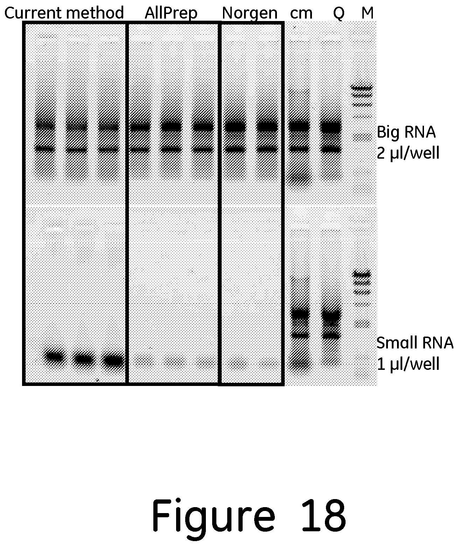

We further compared our protocol with commercial products, in terms of the presence and abundance of small RNA in the isolated total RNA. We choose AllPrep from Qiagen and RNA/DNA/Protein purification kit from Norgen. Both are promoted for simultaneous isolation of genomic DNA, total RNA and proteins. We isolated total RNA using these kits as well as our own protocol. We then attempted to isolate small RNA from the total RNA using the miRNeasy Mini Kit. Results are shown in Figure 18. The small RNA is shown on the bottom panel, while the "large" RNA (total RNA deprived of the small RNA) is on the top panel. Input total RNA from the current method (cm) and the AllPrep kit (Q) are shown as controls. We estimate that total RNA isolated using our protocol contains more than 10% small RNA, while total RNA isolated from other kits contain less than 3% of small RNA.

## Claims

1. A method for the separation and/or purification of at least two cellular components selected from genomic DNA, total RNA and proteins, which method comprising:
a) generating an aqueous solution containing said cellular components by lysing a biological sample with a lysis solution;
b) applying said aqueous solution to a first mineral support under conditions such that genomic DNA binds to the first mineral support;
c) collecting the flowthrough which contains unbound RNA and proteins;
d) mixing said flowthrough from step (c) with a dipolar aprotic solvent selected from Acetone, Acetonitrile, Tetrahydrofuran (THF), Methyl Ethyl Ketone, N,N-Dimethylformamide (DMF), and Dimethyl Sulfoxide to form a mixture, then applying said mixture to a second mineral support under conditions such that RNA binds to said second mineral support; and
e) collecting the flowthrough which contains proteins.

2. The method of claim 1, further comprising washing said first mineral support and eluting the genomic DNA from said first mineral support.

3. The method of claim 1, further comprising washing said second mineral support and eluting the RNA from said second mineral support.

4. The method of claim 1, further comprising purifying the protein from the flowthrough of step (e).

5. The method of claim 1, wherein said lysis solution includes chaotropic salt, non-ionic detergent and reducing agent.

6. The method of claim 5, wherein said chaotropic salt is Guanidine HCl.

7. The method of claim 5, wherein said non-ionic detergent is selected from Triethyleneglycol Monolauryl Ether, (octylphenoxy)Polyethoxyethanol, Sorbitari Monolaurate, T-octylphenoxypolyethoxyethanol, Polysorbate 20 , Polysorbate 40, Polysorbate 60 and Polysorbate 80, or a combination thereof.

8. The method of claim 8, wherein said non-ionic detergent or combination thereof is in the range of 0.1- 10%.

9. The method of claim 1, wherein said lysis solution includes 1- 10 M Guanidine HCl, 0.1-10% TWEEN™ 20 and 0.1- 10% NP-40.

10. The method of claim 1, wherein the first mineral support and the second mineral support are porous or non-porous and comprised of metal oxides or mixed metal oxides, silica gel, silica membrane, glass particles, powdered glass, Quartz, Alumina, Zeolite, Titanium Dioxide, or Zirconium Dioxide.

11. The method of claim 1, wherein the first mineral support and the second mineral support are each silica membranes.

12. A method for isolating microRNA, comprising subjecting the total RNA eluted from claim 3 to one or more additional separation steps to purify the microRNA.

## Patentansprüche

1. Verfahren zur Trennung und/oder Aufreinigung von mindestens zwei zellulären Komponenten ausgewählt aus genomischer DNA, Gesamt-RNA und Proteinen, wobei das Verfahren Folgendes umfasst:
a) Erzeugen einer wässrigen Lösung mit den zellulären Komponenten durch Lysieren einer biologischen Probe mit einer Lyse-Lösung;
b) Aufbringen der wässrigen Lösung auf einen ersten mineralischen Träger unter derartigen Bedingungen, dass genomische DNA an den ersten mineralischen Träger bindet;
c) Sammeln des Durchflusses, welcher ungebundene RNA und Proteine enthält;
d) Mischen des Durchflusses aus Schritt (c) mit einem dipolaren aprotischen Lösungsmittel ausgewählt aus Aceton, Acetonitril, Tetrahydrofuran (THF), Methylethylketon, N,N-Dimethylfolmamid (DMF) und Dimethylsulfoxid zum Bilden einer Mischung, dann Aufbringen der Mischung auf einen zweiten mineralischen Träger unter derartigen Bedingungen, dass RNA an den zweiten mineralischen Träger bindet; und
e) Sammeln des Durchflusses, welcher Proteine enthält.

2. Verfahren nach Anspruch 1, welches ferner das Waschen des ersten mineralischen Trägers und das Eluieren der genomischen DNA von dem ersten mineralischen Träger umfasst.

3. Verfahren nach Anspruch 1, welches ferner das Waschen des zweiten mineralischen Trägers und das Eluieren der RNA von dem zweiten mineralischen Träger umfasst.

4. Verfahren nach Anspruch 1, welches ferner das Aufreinigen des Proteins aus dem Durchfluss von Schritt (e) umfasst.

5. Verfahren nach Anspruch 1, wobei die Lyse-Lösung chaotropisches Salz, nichtionisches Detergens und Reduktionsmittel enthält.

6. Verfahren nach Anspruch 5, wobei das chaotropische Salz Guanidin-HCl ist.

7. Verfahren nach Anspruch 5, wobei das nichtionische Detergens ausgewählt ist aus Triethylenglycol-Monolauryl-Ether, (Octylphenoxy-)Polyethoxyethanol, Sorbitari-Monolaurat, T-Octylphenoxypolyethoxyethanol, Polysorbat 20, Polysorbat 40, Polysorbat 60 und Polysorbat 80 oder einer Kombination davon.

8. Verfahren nach Anspruch 8, wobei das nichtionische Detergens oder die Kombination davon im Bereich von 0,1 bis 10 % liegt.

9. Verfahren nach Anspruch 1, wobei die Lyse-Lösung 1 bis 10 M Guanidin-HCl, 0,1 bis 10 % TWEEN™ 20 und 0,1 bis 10 % NP-40 aufweist.

10. Verfahren nach Anspruch 1, wobei der erste mineralische Träger und der zweite mineralische Träger porös oder nichtporös sind und aus Metalloxiden oder gemischten Metalloxiden, Silicagel, Silicamembran, Glaspartikeln, Pulverglas, Quarz, Aluminiumoxid, Zeolith, Titandioxid oder Zirconiumdioxid bestehen.

11. Verfahren nach Anspruch 1, wobei der erste mineralische Träger und der zweite mineralische Träger jeweils Silicamembranen sind.

12. Verfahren zum Isolieren von microRNA, bei welchem die eluierte Gesamt-RNA aus Anspruch 3 einem oder mehreren weiteren Trennschritten zum Aufreinigen der microRNA unterworfen wird.

## Revendications

1. Procédé de séparation et/ou de purification d'au moins deux composants cellulaires choisis parmi de l'ADN génomique, de l'ARN total et des protéines, ledit procédé comprenant les étapes consistant à :
a) générer une solution aqueuse contenant lesdits composants cellulaires par lyse d'un échantillon biologique avec une solution de lyse ;
b) appliquer ladite solution aqueuse à un premier minéral dans des conditions telles que l'ADN génomique se lie au premier support minéral ;
c) recueillir le flux continu qui contient de l'ARN non lié et des protéines ;
d) mélanger ledit flux continu de l'étape (c) avec un solvant aprotique bipolaire choisi parmi l'acétone, l'acétonitrile, le tétrahydrofuranne (THF), la méthyléthylcétone, le N,N-diméthylformamide (DRF) et le sulfoxyde de diméthyle pour former un mélange, appliquer ensuite ledit mélange à un second support minéral dans des conditions telles que l'ARN se lie audit second support minéral ; et
e) recueillir le flux continu qui contient des protéines.

2. Procédé selon la revendication 1 comprenant en outre le lavage dudit premier support minéral et l'élution de l'ADN génomique dudit premier support minéral.

3. Procédé selon la revendication 1 comprenant en outre le lavage dudit second support minéral et l'élution de l'ARN dudit second support minéral.

4. Procédé selon la revendication 1 comprenant en outre la purification de la protéine du flux continu de l'étape (e).

5. Procédé selon la revendication 1, dans lequel ladite solution de lyse comprend un sel chaotropique, un détergent non ionique et un agent réducteur.

6. Procédé selon la revendication 5, dans lequel ledit sel chaotropique est le guanidine HCl.

7. Procédé selon la revendication 5, dans lequel ledit détergent non ionique est choisi parmi le monolautyléther de triéthylèneglycol, le (octylphénoxy)polyéthoxyéthanol, le monolaurate de sorbitan, le T-octylphénoxypolyéthoxyéthanol, le Polysorbate 20, le Polysorbate 40, le Polysorbate 60 et le Polysorbate 80 où une de leurs combinaisons.

8. Procédé selon la revendication 8, dans lequel ledit détergent non ionique ou une de ses combinaisons se situe dans la plage de 0,1 à 10 %.

9. Procédé selon la revendication 1, dans lequel ladite solution de lyse comprend 1 à 10 M de guanidine HCl, 0,1 à 10 % de TWEEN™ 20 et 0,1 à 10 % de NP-40.

10. Procédé selon la revendication 1, dans lequel le premier support minéral et le second support minéral sont poreux ou non poreux et sont constitués d'oxydes métalliques ou d'oxydes métalliques mixtes, de gel de silice, d'une membrane de silice, de particules de verre, de verre pulvérulent, de quartz, d'alumine, de zéolite, de dioxyde de titane ou de dioxyde de zirconium.

11. Procédé selon la revendication 1, dans lequel le premier support minéral et le second support minéral sont chacun des membranes de silice.

12. Procédé d'isolement de microARN, comprenant la soumission de l'ARN total élué de la revendication 3 à une ou plusieurs étapes de séparation supplémentaires pour purifier le microARN.
